# EUROPEAN PATENT SPECIFICATION

(11) **EP 3 858 403 B1**
(45) Date of publication and mention of the grant of the patent: **31.07.2024**
(21) Application number: 21151632.3
(22) Date of filing: 14.01.2021
(51) Int. Cl.: A61M 5/178, A61M 5/34, A61M 5/32

(54) **MEDICAL DEVICE FOR THE INJECTION OF FLUIDS**
MEDIZINISCHE VORRICHTUNG ZUR INJEKTION VON FLÜSSIGKEITEN
DISPOSITIF MÉDICAL POUR L'INJECTION DE FLUIDES

(30) Priority: 30.01.2020 IT 202000001810
(43) Date of publication of application: 04.08.2021
(73) Proprietor: Molipharma R&D S.r.l., 86100 Campobasso (CB) (IT); Gueli Alletti, Salvatore, 00196 Roma (RM) (IT); Cianci, Stefano, 00168 Roma (RM) (IT)
(72) Inventor: GUELI ALLETTI, Salvatore, 00196 Roma RM (IT); CIANCI, Stefano, 00168 Roma RM (IT); SCAMBIA, Giovanni, 86100 Campobasso CB (IT); UVA, Antonio Emmanuele, 70124 Bari BA (IT)
(74) Representative: Manna, Sara

(56) References cited:
- EP-A2- 0 692 270
- EP-B1- 0 692 270
- WO-A2-2009/107945
- KR-A- 20110 007 512
- KR-Y1- 200 423 638
- US-A1- 2013 211 374
- US-A1- 2018 028 358

## Description

### Technical field of the invention

The present invention relates to a medical device for the delivery of fluids.

In particular, the invention relates to a device for the introduction and/or injection of fluids into a patient's body, and is particularly suitable for cervical injection of a tracking fluid, for diagnostic purposes.

The description below will relate to the specific field of application of gynaecological surgery, nonetheless the invention can be applied in any field related to the injection or delivery of fluids into the human body.

### Background

The treatment of tumours affecting body and neck of the uterus by hysterectomy has undergone an important evolution in the last twenty years. From a typically laparotomy surgery, today in most cases hysterectomy is performed using a minimally invasive approach. Therefore, performing hysterectomy and any additional staging procedures, such as pelvic lymphadenectomy and where appropriate lumboaortic lymphadenectomy, has become common practice.

If, on the one hand, national and international scientific experiences have confirmed the advantages of performing the aforementioned procedures using a minimally invasive approach, on the other hand they have shown procedures such as lymphadenectomy extended to the pelvic stations, and possibly lumboaortic stations, not to have a curative role in the pathology natural history, but only a staging role.

The morbidity (or sick rate) of these procedures - where by morbidity is meant, statistically, the number of disease cases recorded during a predetermined time period in ratio with respect to a predetermined of people considered - exceeds the benefit received by the patient receives. Hence, the need to maintain the diagnostic/staging adequacy of the surgery, reducing its invasiveness.

With regards to the diagnostic aspect, the concept of Sentinel Node Mapping, inherited from other surgical fields, currently represents the solution typically adopted for the morbidity of an extended lymphadenectomy.

The possibility of identifying and removing the first and only drainage lymph node of the uterus (body and/or cervix) allows indeed, on the basis of the most recent scientific experiences, to offer the patient suffering from endometrial or cervical cancer the same diagnostic sufficiency that until a few years ago has been associated with an extensive lymphadenectomy. Thereby, the impact of surgery and the possible complications resulting from this procedure are significantly reduced.

The Sentinel Node Mapping procedure consists in the injection of a tracer, which is usually indocyanine green, at the level of the uterine cervix, at predetermined positions and depths. Generally, the tracer is injected at 3 and 9 o'clock by looking at the cervix from the front, at a depth between 1,5 mm and 15 mm.

The lymphatic drainage of the tracer allows to identify the first drainage lymph node of the organ using a dedicated laparoscopic technology, whose operation is based on the emission of infrared which generates a fluorescence image.

To date, a needle for spinal injection is used for the injection of the tracer, installed on a normal 5 ml disposable syringe. Dimensions and characteristics of the needle are not standardized, as well as the needle-syringe combination is not standardized. Often, during cervical injection, some resistance is encountered due to the parenchymatous characteristics of the cervix itself.

Disadvantageously, the injection of the tracer at the cervix level has considerable difficulties in execution, for the most part due to the low light conditions of the vaginal canal.

Furthermore, the operator is forced to increase the injection pressure at which the tracer is delivered in a narrow space such as the vaginal canal, with the possibility that the increase in the resistance applied to the plunger causes the needle to disengage from the body of the syringe and the solution to exit out of control. Consequently, there would be the risk of losing control over the amount of fluid already injected, and still to be injected.

In view of these criticalities, the injection procedure is currently afflicted by a significant variability in the detection rate of the sentinel lymph node.

In particular, there are some main disadvantages:
- impossibility of measuring the actual depth of injection,
- impossibility of maintaining a constant injection pressure and
- impossibility of accurately dosing the extent of injected fluid.

US2018028358A1 discloses an apparatus for ocular injection including a housing coupled to a medicament container, which is coupled to a needle. An injection assembly is disposed within the housing and includes an energy storage member and an actuation rod. A distal end portion of the actuation rod is disposed within the medicament container. The energy storage member can produce a force on a proximal end portion of the actuation rod sufficient to move the distal end portion of the actuation rod within the medicament container. This can convey at least a portion of a substance from the medicament container via the needle when a distal tip of the needle is disposed within a first region of a target location. The force is insufficient to move the distal end portion of the actuation rod within the medicament container when the distal tip of the needle is disposed within a second region of the target location.

### Summary

The technical problem posed and solved by the present invention is therefore to provide a device which allows to overcome the drawbacks mentioned above with reference to the known art.

The above problem is solved by a device according to claim 1.

Preferred features of the present invention are the subject of the dependent claims.

The device according to the present invention includes a main body operatively associated with a needle for the delivery of the fluid to be injected. The needle is arranged in an adjustable position with respect to the main body, such position being variable by operating dedicated adjustment means, comprised within the main body itself.

Adjusting the position allows to decrease/increase the length of the free end portion of the needle which exits with respect to the main body.

According to a particularly advantageous aspect of the invention, the device further comprises one or more visual reference elements apt to indicate the depth of injection of the needle within the patient's body district, and concurrently further the length of the needle portion exiting with respect to the main body.

In other words, the device of the invention comprises visual reference elements apt to indicate a specific exiting of the needle with respect to the main body.

To allow an adequate display of the injection area, as well as of any visual reference elements, the device can comprise one or more light sources suitably oriented, and more particularly arranged to illuminate at least the free terminal end of the needle together with the aforementioned visual reference elements.

According to a further advantageous aspect, the device allows the operative connection of the needle directly, or by means of an interposed extension element, to an injection device (more simply injector), which bears the fluid to be delivered inside thereof. The device of the invention can comprise injection means configured for the regulated delivery of fluid, preferably provided with additional means for regulating the injection pressure, in order to achieve the necessary injection pressure values and to control the extent of injected fluid with high precision. Such means can have the body structure of a known type of syringe, preferably a pistol syringe or a syringe with screw piston, or it can comprise an electrically operating pressurization device.

This advantageous configuration allows to achieve a standardized delivery pressure, together with a controlled volumetric dosage of the injected fluid.

Therefore, by means of preferred embodiments of the device of the invention, a standardization of the delivery parameters of tracking fluids (extent and speed of delivery of the fluid) at the level of the cervix can be achieved, significantly reducing the operator-dependent variables by improving the detection rate of the sentinel lymph node.

Advantageously, the variants providing the combination of controlled injection means and visual reference elements subjected to targeted lighting give the device a high precision of use, both with respect to the extent of the delivered fluid and with respect to the positioning of the injection point of the fluid in the patient.

A further advantage of the device of the invention is that it can be used for the delivery of fluid on several occasions, without encountering the inconvenience that undesired fluid delivery occurs between one delivery and the next.

Other advantages, characteristics and methods of use of the present invention will become apparent from the following detailed description of several embodiments, presented by way of non-limiting example.

### Brief description of the figures

Reference will be made to the Figures of the attached drawings, in which:
- Figure 1 shows an axonometric view, in semi-transparency, of a first preferred embodiment of a device according to the present invention;
- Figure 2 shows a side view, in exploded configuration, of the device of Figure 1;
- Figures 3, 4 and 5 respectively show a side view of a first, second and third preferred embodiment of the device of the invention.

The aforementioned attached Figures are to be considered purely by way of non-limiting example.

### Detailed description of preferred embodiments of the invention

The subject of the present invention is a medical device for the injection of a fluid into a patient's body, or into a cavity thereof.

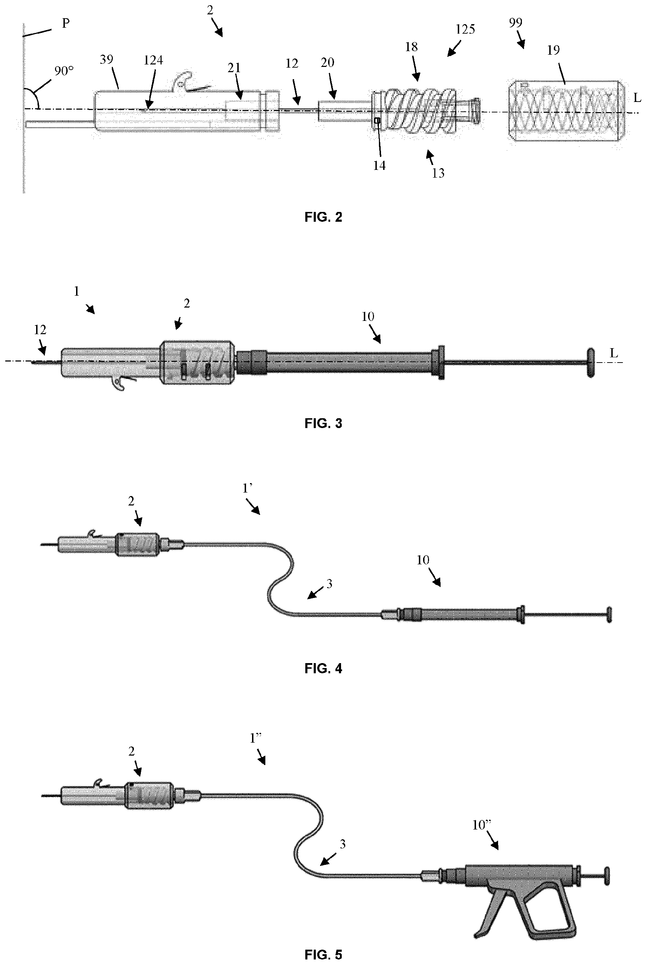

With reference to Figure 3, a first preferred embodiment of the device according to the present invention is overall denoted by 1.

Device 1 comprises an injector 10 of fluid to be delivered to a patient, a main body 2 and a needle 12 for the delivery of the fluid itself.

The main body 2 is preferably made of transparent material, and is configured to house the aforementioned needle 12.

According to a first preferred embodiment of the invention, the main body 2 is shaped as a tubular body, which is preferably hollow and preferably has an elongated conformation, extending according to a longitudinal development direction L, which is preferably the same direction of extension of the needle 12. Preferably, the overall configuration of the main body 2 is cylindrical and even more preferably symmetrical with respect to an axis of longitudinal symmetry, which coincides according to this embodiment with the longitudinal development direction L.

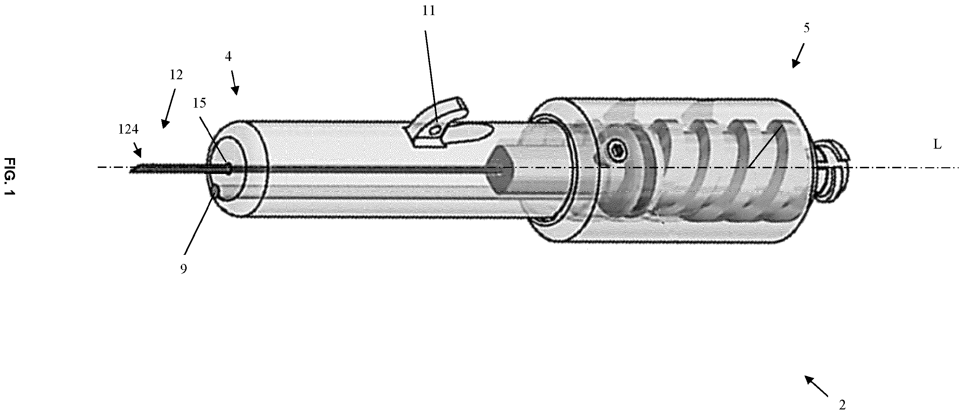

According to such a configuration of the main body 2, as shown in more detail in Figures 1 and 2, a distal end 4 and a proximal end 5 of the main body 2 may be identified. The proximal end 5 is intended to be, during use, the end of the main body 2 closest to the user, while the distal end 4 is intended to be, during use, the furthest away from the user and therefore the closest to the injection site.

During use, the distal end 4 can be placed in direct contact with the patient's body district at the point in which the injection is to be performed. In particular, before the delivery, the needle 12 may be provided to be fully retracted within the main body 2, and to be extracted with respect to the latter only once the distal end 4 of the main body 2 has been in contact with the body district or injection site.

With particular reference to Figure 2, the injection site is represented by way of example as a distal plane P, with respect to which the device 1, in use, is arranged orthogonally during the delivery of the fluid.

The needle 12 is operatively connected to the main body 2 at the distal end 4. In particular, the main body 2 comprises at the distal end 4 a seat or channel 15, shaped as a through hole, apt to allow the sliding of the needle 12. The sliding of the needle 12 is preferably made according to the longitudinal direction L. The seat 15 is preferably centered with respect to the distal end 4, and is preferably aligned along the axis of symmetry L.

In particular, the main body 2 is configured to house at least partially, more preferably entirely, the needle 12 there inside.

Furthermore, the main body 2 comprises means for adjusting the position 99 of the needle 12, selectively operable.

Such means for adjusting the position 99 is configured to allow a movement of the needle 12 according to a sliding motion with respect to the main body 2, which will be better described below.

Such motion provides a sliding or translation direction of the needle 12 parallel to, or coinciding with, the longitudinal development direction L. Due to this configuration, the length of the portion of needle 12 exiting with respect to the main body 2 can be increased/decreased.

Preferably, the seat 15 is configured to realize a guide for the correct sliding of the needle 12 with respect to the main body 2, avoiding undesired bending or deviations of the needle 12 with respect to the longitudinal sliding direction L.

The configuration of device 1 is such that a sliding motion towards the proximal end 4 of the needle 12 determines an increase in the portion of needle 12 which exits from the main body 2 through the seat 15, while a sliding motion of the needle 12 towards the distal end 5 determines a decrease in the portion of needle 12 which exits from the main body 2 through the seat 15.

In particular, the sliding of the needle 12 with respect to the main body 2 is achieved due to the coupling of the aforementioned means for adjusting the position 99 comprised in the main body 2 with respective guide means 13 comprised in the needle 12.

According to the preferred embodiment of the invention shown in Figures 1 and 2, the means for adjusting the position 99 comprises a ring nut 19 having a threaded hole inside, like a nut screw.

Correspondingly, the guide means 13 of the needle 12 is configured to engage with the aforementioned ring nut, and comprises a first guide element 18 for this purpose. The first guide element 18 is preferably configured as a threaded element keyed onto the rear end 125 opposite to the free end (or tip) 124 of the needle 12.

The first guide element 18 is configured to engage the threaded hole of the ring nut 19, in such a way as to allow the ring nut 19 to rotate thereon.

Alternatively, the ring nut 19 can be associated with a motion conversion mechanism, which transforms the rotary motion of the ring nut 19 into a translational motion of the needle 12.

According to a preferred variant of the invention, the needle 12 has at the rear terminal end 125, in a more advanced position towards the tip 124 with respect to the first guide element 18, an enlarged portion with a prismatic profile (denoted by the reference number 20 in Figure 2), which creates a second guide element 20.

Preferably, the needle 12 comprising the first guide element 18 and/or the second guide element 20 is made of a single piece.

The second guide element 20 therefore has a polygonal section, which is larger than the circular section of the remaining portion of needle 12. Such second guide element 20 is comprised in the aforementioned guide means 13.

Correspondingly, at the internal surface of a remaining support portion 39 of the main body 2, a seat or prismatic slot 21, with a geometry complementary to that of the second guide element 20 and intended to engage with the latter, or rather to house the same, is obtained. Preferably, the prismatic slot 21 is arranged in a centered position with respect to the main body 2, and develops according to the longitudinal direction L.

The support portion 39 is coupled to the means for adjusting 99, in particular to the ring nut 19. Even more preferably, the ring nut 19 is integral with the support portion 39 as regards the axial translation motion, although it is free to rotate with respect to the same.

According to such configuration, the mutual motion between the second guide element 20 and the prismatic seat 21 can only be of pure translation.

According to the preferred embodiment still shown in Figure 2, the ring nut 19 can be rotated, preferably around the longitudinal axis L, to obtain the needle 12 to slide with respect to the main body 2. At the same time, the needle 12 will result in a position more axially distant/near, along the longitudinal direction L, with respect to the terminal end 4 of the main body 2.

According to this actuation of the means for adjusting the position 99, the depth of penetration of the free end 124 of the needle 12 can be modified with respect to the surface of the patient's body district in which to deliver the fluid (distal plane P).

It should be understood that, in particular, the achieved advancement/retraction of the needle 12 with respect to the main body 2 is proportional to a predetermined angle of rotation of the ring nut 19.

By way of example, the device 1 can be configured in such a way as to correspond, at predetermined angles of rotation of the ring nut 19 equal respectively to 90° and 180° with respect to an initial position of the same, to a linear advancement of the needle 12, exiting from main body 2, respectively equal to about 3 and 15 mm.

The locking of the guides means 13, and therefore of the needle 12 integral therewith, in a fixed position (continuously, or discrete positions) with respect to the main body 2 can be obtained by simple friction, or by means of teeth or notches provided in the ring nut 19 itself, or by rebating the first guide element 18 on an end stop pre-arranged on the internal surface of the main body 2.

With reference to Figure 1, in case of fluid delivery at the uterine cervix, the main body 2 can be kept in a stable position during injection by means of a retention element 11 borne on its external surface, which can be configured as a hook, or bear a buttonhole or eyelet, in order to be fixed to the exo-cervix by means of Hegar forceps or surgical wire.

The device according to the invention can further provide the presence of one or more visual reference elements 14 arranged in order to indicate the depth the needle 12 exits from the main body 2, i.e. the distance of the distal end 124 of the needle 12 from the plane distal P of the user's body district, or the depth the needle 12 penetrates into the same. Such reference elements 14 can be graphic symbols, coloured and/or fluorescent bands.

Such visual reference elements 14 can be borne, for example, on the means guides 13, in the form of one or more symbols or coloured bands visible through suitable windows obtained on the surface of the ring nut 19. Thereby, during use, the operator can get accurate information regarding the position of the tip of the needle 12 with respect to the patient's body, knowing exactly how much it has penetrated there inside, without requiring to directly observe the needle 12. Alternatively, or additionally, visual reference elements can be arranged on the external lateral surface of the needle 12 intended to exit from the main body 2.

According to a preferred embodiment of the invention, there are a first window and a second window (or openings) on the ring nut 19 surface, configured to realize a reference visible by the operator at needle leakage with respect to the main body 2 respectively equal to 3 mm and 15 mm.

Furthermore, the device 1 preferably comprises a lighting system 9 apt to illuminate the injection area and/or the visual reference elements 14, in order to allow an adequate and complete visualization.

A preferred application of device 1 is indeed in gynaecological surgery, for the SLNM (Sentinel Lymph Node Mapping) procedure; in this surgery, the injection takes place in a cavity of the patient's body where a clear vision of the operating field is rather difficult to be achieved.

Preferably, the lighting system 9 is oriented in such a way as to emit light at the visual reference elements 14. The light emitted can comprise radiations such as to allow the exhibition of fluorescence phenomena of the visual reference elements 14, which will comprise fluorescent paints. For this purpose, a preferred embodiment of the lighting system 9 can be configured as an elongated light element, arranged within the main body 2 at the windows to visualize the visual reference elements 14 and oriented towards the free end 124 of the needle 12, in order to allow appropriate lighting of both the visual reference elements 14 and the injection site. In particular, the light element can be arranged with a terminal end exiting at the distal end 4 of the main body 2.

According to a preferred embodiment, the lighting system 9 can be integrated inside the main body 2, or the guide means 13, or the ring nut 19.

Furthermore, the device 1 can comprise the housing of a power supply battery, apt to supply energy to the lighting system 9. The power supply battery can be housed in the main body 2, preferably in the support portion 39, and can be of any type commonly used commercially. Alternatively, an electrical supply associated with a chemical reaction can be provided.

Furthermore, the main body 2 and/or the ring nut 19 can have an anti-slip surface (for example milled, or with rubbery material inserts) to ensure optimal grip by the operator.

Alternatively, the device can provide a grip element at the main body surface 2, made integral with the main body 2 or as a separate element, selectively connectable to the body 2.

To allow the fluid delivery, the needle 12 is fluidly connected to the injector 10 by means of a sealed connection. According to the embodiment of Figure 3, the main body 2 is made integral with the injector 10 by means of a direct mechanical connection, in particular a sealed connection (for example Luer Lock cone).

Further embodiments, object of Figures 4 and 5, provide instead the main body 2 and the injector 10 to be not integral with each other, and the fluidic connection between the syringe 12 and the injector 10 to be realized by means of a connecting element 3 interposed therebetween, preferably flexible. The connecting element 3 is in particular a tube of extension.

According to such variants, the injector 10 can be dislocated in a remote position with respect to the main body 2. This is particularly advantageous in case the injection is to be carried out at patient's body district which are not easily accessible, facilitating the operation of dosing the injected fluid possibly performed by an operator other than the operator manipulating the main body 2 at the patient's body district. Furthermore, the presence of the connecting element 3 allows to handle the device 1 with greater ease, avoiding accidental extraction of the needle.

The injector 10 can be a normal portion of a syringe, comprising a fluid reservoir and a plunger for its injection. Alternatively, in order to achieve the injection pressures required for delivery and to better control the amount of injected fluid, the injector can comprise a screw piston, or be shaped like a pistol syringe (Figure 5). Furthermore, an injector 10 can be provided comprising an electrically operating pressurization device.

Such configurations allow to achieve a more standardized fluid delivery pressure and a controlled volumetric dosage. This is particularly useful in particular situations, such as cervical injection: the parenchymatous characteristics of the cervix itself give rise to a certain resistance and the operator can, thereby, perform a standardized injection. For such a specific type of application, the injector 10 is configured to contain a volume of about 5 ml and the fluid used can be a solution of indocyanine green (ICG, IndoCyanine Green).

The present invention has been hitherto described with reference to preferred embodiments. It should to be understood that there may be other embodiments belonging to the same inventive core, as defined by the scope of the claims set forth below.

## Claims

1. Medical device (1) for the injection of a fluid into a patient's body, said device (1) comprising:
- an injector (10), comprising a reservoir of the fluid to be injected,
- a needle (12) comprising a free end (124) for the delivery of the fluid and being fluidically connected to said injector (10),
- a hollow tubular main body (2) extending according to a longitudinal development direction (L) and configured to house said needle (12) inside thereof,
said main body (2) comprising means for adjusting the position (99) of said needle (12) with respect to said main body (2),
which means for adjusting the position (99) is configured to engage with respective guide means (13) of said needle (12) and selectively operable to allow a sliding of said needle (12) with respect to said main body (2) according to a direction parallel to, or coinciding with, said longitudinal development direction (L), in such a way as to increase/decrease the length of the portion of needle (12) exiting with respect to said main body (2),
wherein said guide means (13) comprises a second guide element (20) at a rear end (125) of said needle (12) opposite to said free end (124), said second guide element (20) having a prismatic profile and
wherein said main body (2) comprises a support portion (39) coupled to said means for adjusting the position (99), wherein said support portion (39) comprises a prismatic seat (21) having a geometry complementary to that of said second guide element (20) for housing the latter inside thereof,
wherein said means for adjusting the position (99) comprises a ring nut element (19) which comprises a threaded hole configured to engage with said guide means (13), and
said guide means (13) comprises a first guide element (18) configured as a threaded element, keyed onto said needle (12) at a rear end (125) thereof opposite to said free end (124), wherein said first guide element (18) is configured to engage with said threaded hole of said ring nut (19),
wherein said needle (12) comprising said first and second guide elements (18, 20) is made of a single piece.

2. Device (1) according to the preceding claim, wherein said ring nut (19) comprises one or more visual reference elements (14) apt to indicate the extent of the exiting of said needle (12) with respect to said main body (2), wherein said one or more visual reference elements (14) are preferably made in the form of coloured and/or fluorescent bands.

3. Device (1) according to the preceding claim, wherein said main body (2) comprises a lighting system (9) apt to illuminate a free end (124) of said needle (12) and/or said one or more visual reference elements (14).

4. Device (1) according to one of preceding claims, wherein said injector (10) is fluidically connected to said needle (12) by means of a connecting element (3) interposed therebetween, said connecting element (3) being a flexible tube of extension.

5. Device (1) according to one of preceding claims, wherein said injector (10) comprises a screw piston or an electrically operating fluid pressurization device.

6. Device (1) according to one of preceding claims, wherein said main body (2) is made of transparent material.

## Patentansprüche

1. Medizinische Vorrichtung (1) für eine Injektion eines Fluids in einen Körper eines Patienten, die Vorrichtung (1) umfassend:
- einen Injektor (10), umfassend ein Reservoir des Fluids, das injiziert werden soll,
- eine Nadel (12), umfassend ein freies Ende (124) für die Abgabe des Fluids und die mit dem Injektor (10) fluidisch verbunden ist,
- einen hohlen, schlauchförmigen Hauptkörper (2), der sich entlang einer Längsentwicklungsrichtung (L) erstreckt und konfiguriert ist, um die Nadel (12) im Inneren davon aufzunehmen,
der Hauptkörper (2) umfassend ein Mittel zum Einstellen der Position (99) der Nadel (12) hinsichtlich des Hauptkörpers (2),
wobei das Mittel zum Einstellen der Position (99) konfiguriert ist, um mit dem jeweiligen Führungsmittel (13) der Nadel (12) in Eingriff zu stehen und selektiv betätigbar ist, um ein Gleiten der Nadel (12) hinsichtlich des Hauptkörpers (2) gemäß einer Richtung zu ermöglichen, die parallel zu der Längsentwicklungsrichtung (L) verläuft oder mit dieser zusammenfällt, derart, dass die Länge des Abschnitts der Nadel (12), der hinsichtlich des Hauptkörpers (2) austritt, vergrößert/verkleinert wird,
wobei das Führungsmittel (13) ein zweites Führungselement (20) an einem hinteren Ende (125) der Nadel (12) gegenüber dem freien Ende (124) umfasst, wobei das zweite Führungselement (20) ein prismatisches Profil aufweist und
wobei der Hauptkörper (2) einen Trägerabschnitt (39) umfasst, der mit dem Mittel zum Einstellen der Position (99) gekoppelt ist, wobei der Trägerabschnitt (39) einen prismatischen Sitz (21) umfasst, der eine Geometrie aufweist, die zu der des zweiten Führungselements (20) zum Aufnehmen des Letzteren im Inneren davon komplementär ist,
wobei das Mittel zum Einstellen der Position (99) ein Ringmutterelement (19) umfasst, das ein Gewindeloch umfasst, das konfiguriert ist, um mit dem Führungsmittel (13) in Eingriff zu stehen, und
das Führungsmittel (13) ein erstes Führungselement (18) umfasst, das als ein Gewindeelement konfiguriert ist, das auf die Nadel (12) an einem hinteren Ende (125) davon gegenüber dem freien Ende (124) verkeilt ist, wobei das erste Führungselement (18) konfiguriert ist, um mit dem Gewindeloch der Ringmutter (19) Eingriff zu stehen,
wobei die Nadel (12), umfassend das erste und das zweite Führungselement (18, 20), aus einem einzigen Stück hergestellt ist.

2. Vorrichtung (1) nach dem vorstehenden Anspruch, wobei die Ringmutter (19) ein oder mehrere visuelle Referenzelemente (14) umfasst, die geeignet sind, um das Ausmaß des Austretens der Nadel (12) hinsichtlich des Hauptkörpers (2) anzuzeigen, wobei das eine oder die mehreren visuellen Referenzelemente (14) vorzugsweise in Form farbiger und/oder fluoreszierender Bänder hergestellt sind.

3. Vorrichtung (1) nach dem vorstehenden Anspruch, wobei der Hauptkörper (2) ein Beleuchtungssystem (9) umfasst, das geeignet ist, um ein freies Ende (124) der Nadel (12) und/oder das eine oder die mehreren visuellen Referenzelemente (14) zu beleuchten.

4. Vorrichtung (1) nach einem der vorstehenden Ansprüche, wobei der Injektor (10) mittels eines Verbindungselements (3), das dazwischen angeordnet ist, mit der Nadel (12) fluidisch verbunden ist, wobei das Verbindungselement (3) ein flexibler Verlängerungsschlauch ist.

5. Vorrichtung (1) nach einem der vorstehenden Ansprüche, wobei der Injektor (10) einen Schraubkolben oder eine elektrisch betriebene Fluiddruckbeaufschlagungsvorrichtung umfasst.

6. Vorrichtung (1) nach einem der vorstehenden Ansprüche, wobei der Hauptkörper (2) aus transparentem Material hergestellt ist.

## Revendications

1. Dispositif médical (1) destiné à l'injection d'un fluide dans le corps d'un patient, ledit dispositif (1) comprenant :
- un injecteur (10), comprenant un réservoir du fluide à injecter,
- une aiguille (12) comprenant une extrémité libre (124) pour la distribution du fluide et étant raccordée fluidiquement audit injecteur (10),
- un corps principal (2) tubulaire creux s'étendant selon une direction de développement longitudinal (L) et configuré pour loger ladite aiguille (12) à l'intérieur de celui-ci,
ledit corps principal (2) comprenant un moyen permettant d'ajuster la position (99) de ladite aiguille (12) par rapport audit corps principal (2),
le moyen permettant d'ajuster la position (99) étant conçu pour venir en prise avec un moyen de guidage (13) respectif de ladite aiguille (12) et sélectivement fonctionnel pour permettre un coulissement de ladite aiguille (12) par rapport audit corps principal (2) selon une direction parallèle à, ou coïncidant avec, ladite direction de développement longitudinal (L), d'une manière telle à augmenter/diminuer la longueur de la partie de l'aiguille (12) sortant par rapport audit corps principal (2),
dans lequel ledit moyen de guidage (13) comprend un second élément de guidage (20) au niveau d'une extrémité arrière (125) de ladite aiguille (12) opposée à ladite extrémité libre (124), ledit second élément de guidage (20) ayant un profil prismatique et
dans lequel ledit corps principal (2) comprend une partie de support (39) accouplée audit moyen permettant d'ajuster la position (99), dans lequel ladite partie de support (39) comprend un logement prismatique (21) ayant une géométrie complémentaire à celle dudit second élément de guidage (20) permettant de loger ce dernier à l'intérieur de celui-ci,
dans lequel ledit moyen permettant d'ajuster la position (99) comprend un élément d'écrou circulaire (19) qui comprend un trou fileté conçu pour venir en prise avec ledit moyen de guidage (13), et
ledit moyen de guidage (13) comprend un premier élément de guidage (18) conçu en tant qu'élément fileté, claveté sur ladite aiguille (12) au niveau d'une extrémité arrière (125) de celle-ci opposée à ladite extrémité libre (124), dans lequel ledit premier élément de guidage (18) est conçu pour venir en prise avec ledit trou fileté dudit écrou circulaire (19),
dans lequel ladite aiguille (12) comprenant lesdits premier et second éléments de guidage (18, 20) est constituée d'une pièce unique.

2. Dispositif (1) selon la revendication précédente, dans lequel ledit écrou circulaire (19) comprend un ou plusieurs éléments de référence visuelle (14) aptes à indiquer l'étendue de la sortie de ladite aiguille (12) par rapport audit corps principal (2), dans lequel ledit ou lesdits éléments de référence visuelle (14) sont de préférence fabriqués sous la forme de bandes colorées et/ou fluorescentes.

3. Dispositif (1) selon la revendication précédente, dans lequel ledit corps principal (2) comprend un système d'éclairage (9) apte à illuminer une extrémité libre (124) de ladite aiguille (12) et/ou ledit ou lesdits éléments de référence visuelle (14).

4. Dispositif (1) selon l'une des revendications précédentes, dans lequel ledit injecteur (10) est raccordé fluidiquement à ladite aiguille (12) au moyen d'un élément de raccord (3) intercalé entre eux, ledit élément de raccord (3) étant un tube d'extension flexible.

5. Dispositif (1) selon l'une des revendications précédentes, dans lequel ledit injecteur (10) comprend un piston à vis ou un dispositif de mise sous pression de fluide actionné électriquement.

6. Dispositif (1) selon l'une des revendications précédentes, dans lequel ledit corps principal (2) est constitué de matériau transparent.
